# EUROPEAN PATENT APPLICATION

(11) **EP 1 358 859 A1**
(43) Date of publication of application: **05.11.2003**
(21) Application number: 02425272.8
(22) Date of filing: 29.04.2002
(51) Int. Cl.: A61F 2/30, A61L 27/32, A61C 8/00

(54) **Bone prostheses having multilayer interface**

(71) Applicant: Politecnico Di Milano, 20133 Milano (IT)
(72) Inventor: Montevecchi, Franco Maria, 20100 Milan (IT); Mantero, Sara, 20100 Milan (IT); Redaelli, Alberto, 20100 Milan (IT); Soncini, Monica, 20100 Milan (IT)
(74) Representative: Gislon, Gabriele

(57) **Abstract**

A bone prosthesis has a portion of interface with the bone which comprises a porous layer (2) with cavities (3, 4) having prefixed and reproducible dimensions and arrangement, and at least an osteoinductive layer containing one or more chemical compounds that can stimulate and promote the growth of bone tissue.

## Description

The present invention relates to bone prostheses, in particular to improved orthopedic and dental prostheses with improved multi-layer interface.

More particularly, the invention relates to metal bone prostheses. Two types of methods for fixing bone prostheses (osteoprostheses) to the related bone are known in the art: fixing by cementing and fixing without cementing.

Fixing the prosthesis by cementing allows reduced recovery times, but has the drawback that the so fixed prosthesis sustains blows poorly, and is therefore insufficiently suitable to allow bearers to carry out physical activities in the long run. Prosthesis fixing techniques have been therefore developed that do not require cementing.

A known type of non-cemented prosthesis has smooth surfaces that are mechanically anchored to the bone by pressure. However, this type of prosthesis is subject to mobility at the interface and has a reduced resistance to torsion and tangential stresses. In order to solve these problems, non-cemented prostheses have been developed with a coat that becomes integral with the bone tissue and allows cellular proliferation and integration with the surrounding bone tissue.

The materials presently used for this purpose comprise porous coats an the so-called osteoinductive coats. In metal prostheses, porous coats are usually made from the same metal as the prosthesis, generally titanium, titanium alloys or tantalum, and are realized either by applying on the surface of the prosthesis microspheres or metal fibers or by adopting technologies for modifying the surface with plasma treatments. The so obtained surface has a porosity that must be adequate to allow the regeneration of the bone tissue within it and therefore the anchoring of the bone to the prosthesis.

Osteinductive coats are generally realized with calcium phosphates, in particular hydroxyapathite. These materials are chemically very similar to the mineral part of the bone, and in this manner a condition can be obtained wherein a bone apposition occurs between the surface of the implant and the bone to which it is fixed, without interposition of fibrous tissue.

The drawback of these techniques lies in that rehabilitation times are long (in the order of months) and they do not solve entirely always and in any conditions the problems of fixing the implant, both in the short and the long run.

Another problem generalized for bone prostheses is their life expectancy: in particular for total hip bone prostheses (coxo-femoral prostheses) life expectation is of about fifteen years on the average. This limitation is not due to a progressive deterioration of the prosthesis, which in fact maintains adequate characteristics for periods much longer than the bearer's life, but rather to the onset, at the interface between the prosthesis and the bone tissue, of phlogosis phenomena associated to the growth of fibrous tissue that, by replacing bone tissue, cannot ensure any longer the integration and the mechanical resistance necessary for the transmission of stresses. As generally no more than two interventions can be performed on the same femur, this results in the impossibility of ensuring a lasting use of the prosthesis to those who have undergone a first intervention at a relatively young age.

Object of the present invention is to provide bone prostheses having a multi-layer interface with optimum characteristics of mechanical resistance against stresses and such as to result in an improved and more stable fixing of the implant to the bone so as to obtain a prostheses' life that is longer than with prostheses of the known art.

This goal is achieved by the present invention which relates to a bone prosthesis characterized in that the interface with the bone comprises a porous layer and at least an osteoinductive layer containing one or more chemical compositions that can stimulate and promote the growth of bone tissue.

A further object of the present invention is a process for the production of a bone prothesis having a multi-layer interface of the above mentioned type, characterized in that it comprises the following steps:
- providing a porous layer on the interface surface between the prosthesis and the bone;
- providing at least one osteoinductive layer comprising one or more chemical compositions that can stimulate and promote the adhesion and growth of bone tissue on said porous layer.

According to a preferred embodiment there are provided a porous layer, a hydroxyapathite layer - possibly integrated with chemical and biochemical substances or compositions (typically but not exclusively comprising growth factors) and a layer of drugs and chemical and biochemical compounds typically but not exclusively comprising adhesion factors.

According to another embodiment, the porous layer is composed of a plurality of cavities obtained by mechanical operations (laser, electron beam, drilling, punching), that are characterized by prefixed dimensions and arrangement rather than a casual one as happens in the known art embodiments.

The invention provides many advantages.

In fact, the adoption of a porous and in particular micro-porous layer combined with an hydroxyapathite layer and possibly a biomolecular layer allows to stimulate the growth of the bone tissue; the outermost layer comprising drugs and adhesion factors prevents phlogosis phenomena and sends biological signals to "attract" the cells that activate the growth of the bone tissue, while the innermost "disposable" layer from hydroxyapathite (which is reabsorbed and reshaped by bone tissue) allows to consolidate the growth so started to eventually generate a strong anchoring to the prosthesis porous surface, the whole within a time shorter than those provided by the known art for non-cemented bone prostheses.

A further advantage lies in that by making cavities by means of "mechanical" operations, i.e. laser, electron beam, drilling or punching, it is possible to obtain cavities having dimensions and an arrangement that are not casual but designed, reproducible and prefixed, allowing thereby to obtain a bone-prosthesis anchoring that is much safer and more reproducible than those of the known art.

Differently from the application of micro-spheres and metal wires, this type of operation can, in fact, satisfy precise criteria of reproducibility quality control; these workings, in fact, are planned with CAM (computer assisted manufacturing) systems by which a working precision is reached in the order of a few microns.

Besides, the porous, or better the micro-porous, layer of the prosthesis surface has a structure having dimensions of from 50 to a few hundred microns, i.e. dimensions that are compatible with those of the substructures of the bone tissue to be integrated with the prosthesis.

The invention will be now described in greater detail with reference to the drawings attached by way of illustrative non limiting examples, wherein:
- Figure 1 is a cross-section view of a detail of the prosthesis surface according to a preferred embodiment of the invention;
- Figure 2 is an enlarged view of a detail of Figure 1;
- Figures 3 and 4 are a cross-section view of embodiments of the porous layer according to the invention; and
- Figure 5 is a schematic perspective view of a portion of the interface according to the invention.

The prosthesis according to the present invention will be described with reference to the commonly utilized prostheses, i.e. prostheses made from metal, generally titanium or titanium alloys, tantalum and like metals and alloys suitable for the purpose. However, this must not be construed as limiting the protection scope to metal prostheses only, as the invention applies to the prostheses independently on the material which they are made from.

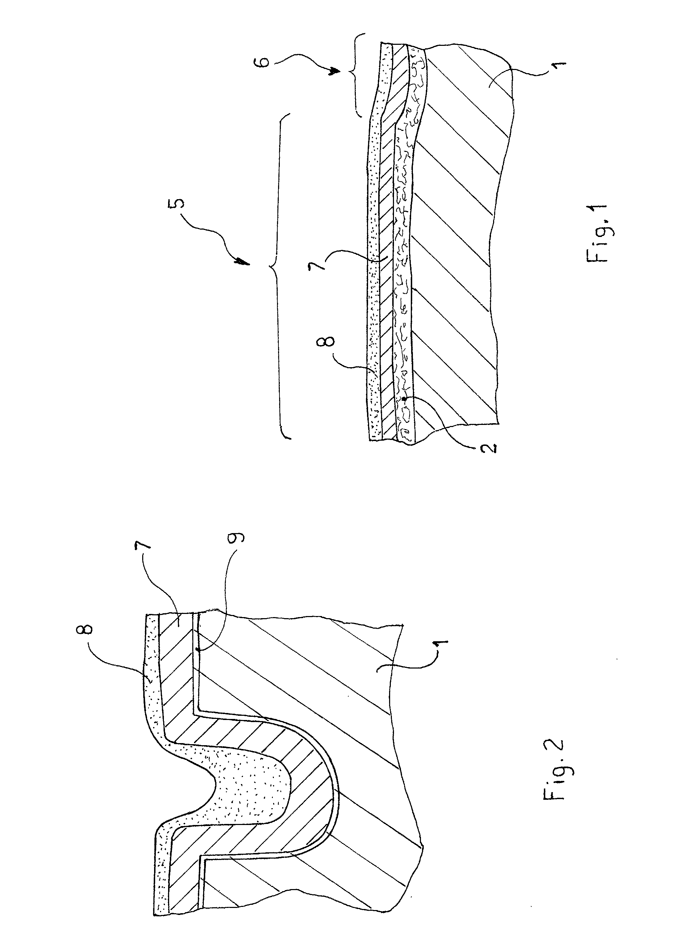

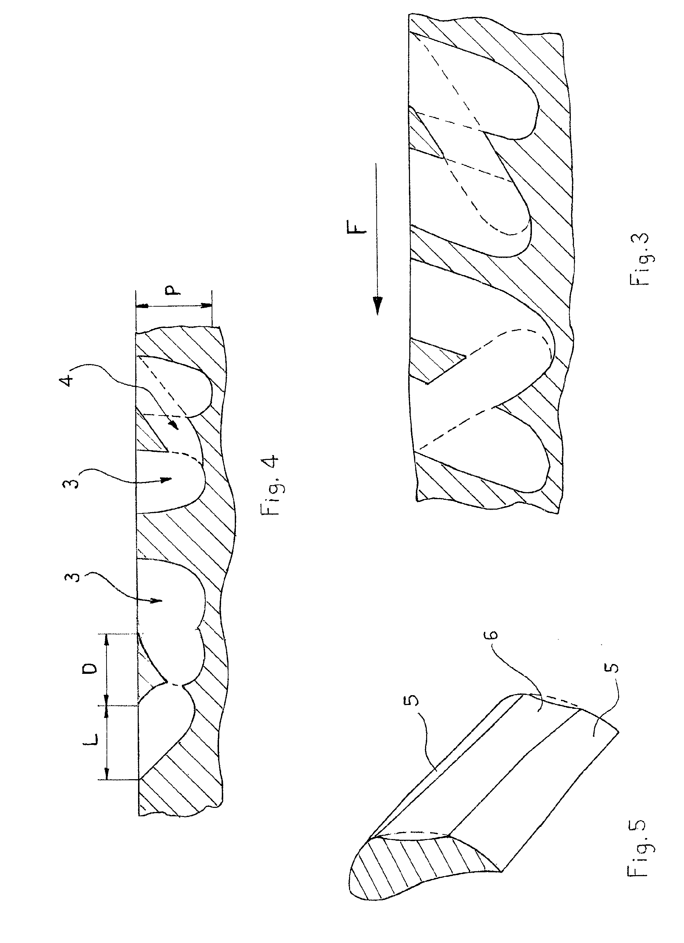

With reference to Figure 1, body 1 of the prosthesis shows a porous surface layer 2, namely an irregular and rough surface wherein a plurality of cavities is obtained. Such surfaces are known in the art and are obtained by means of application of metal wires, micro-spheres or plasma surface treatment; additionally to these techniques, metal foams and plasma spray processes can be used to obtain layer 2. In the preferred embodiment of the invention, the porous surface 2 is obtained by laser or electron beam workings or also with more traditional workings of blind drilling or punching, and is comprising (Figs. 3 and 4) a plurality of cavities 3 that can be in communication with each other by means of passage holes 4, as can be seen in Figures 3 and 4. The advantage provided by the adoption of cavities obtained by mechanical working (wherein by the term "mechanical working" it is meant to indicate also and especially laser or electron beam machining or other equivalent method or also more traditional workings of blind drilling or punching) lies in that it is possible to obtain cavities having known and prefixed dimensions and arrangement, and such as to have in different zones of the interface surface characteristics pre-established for the application. In other words, the width L of the cavity, its distance D from the nearer cavity and its depth P are values that are not subject to random statistic variations, as happens instead with the techniques of the known art, but are selected and fixed as functions of the type of prosthesis, interface zones and, above all, they are the same for all the prostheses of a same type.

Preferably, the width (i.e. the diameter) L is comprised in the interval between 50 and 500 microns and more preferably between 200 and 400 micron; the depth P of the cavity is comprised between 200 and 800 microns and more preferably between 400 and 600 microns; the distance D is preferably comprised between the proportions 0.5L and 1.5L, wherein L is the width of the cavity.

Obtaining the porous surface through mechanically worked cavities (in the aforesaid meaning) also allows to choose the inclination of the cavity axis and to prefix such value as function, for instance, of the interface zone on the prosthesis. In this manner, in some areas, angle α between the perpendicular to the surface and the cavity axis will be minor or non-existing, while in other areas of the interface, angle α will be greater. The inclination of the cavity axis shall be such as to facilitate the insertion of the prosthesis in the bone, minimizing thereby the damage to the latter and the possible interface layers. Figure 3 shows cavity inclinations suitable for the direction according to which the prosthesis insertion movement takes place with respect to the bone (arrow F); these inclinations that cause cavities to be oriented in a direction contrary to the direction of arrow F, appear to be important in the surface portions wherein the prosthesis and the bone are in close touch with each other during the insertion of said prosthesis, but not in the surface portions wherein the prosthesis and the bone are not in close touch during the insertion.

In an embodiment of the invention, schematized in partial view in Figure 5, the part of prosthesis that is inserted in the bone shows surface portions 5 that are forced against the bone and portions 6 that are so shaped as to engage the bone to a minimum extent or not at all; for instance, the surface portions 6 that are not forced against the bone are lower than the rest of the surface 5 of the prosthesis by a value in the order of 5-30 microns. As mentioned above, the orientation and the arrangement, as well as the shape, of the cavities obtained on surfaces 5 and 6 may be different. Preferably, the arrangement in zone 5 will be inclined, as the one shown in Figure 3, while in zone 6, the arrangement will be like the one shown in Figure 4; in any case, the distance between the lower areas 6 and the bone is never such as to hinder the attraction and stimulation action of the osteoinductive layer that is present at the interface, as described below.

Going back now to Figure 1, the interface of the prosthesis comprises also at least a layer from osteoinductive material, i.e. a layer comprising, or consisting of, hydroxyapathite, phosphates, to which chemical and biochemical compositions (typically but not exclusively comprising growth factors) have been possibly and preferably added to stimulate and promote the growth of the bone tissue and obtain an integration between the bone and the prosthesis by means of growth of bone tissue and its anchoring in the cavities of the porous layer 2.

In the preferred embodiment shown, the osteinductive layer is constituted of two layers: a first layer 7, which is formed by or contains calcium phosphates and preferably hydroxyapathite, and a second layer 8 containing drugs and chemical and biochemical compositions, typically but not exclusively adhesion factors.

Preferably, Ca, Mg and P salts and other salts are mixed with the hydroxyapathite of the first layer 7, in order to provide support to growth, as well as drugs, for instance slow release antibiotics protected by microcontainers (for instance, microcapsules). Besides, other chemical or biochemical compositions that can promote bone growth, for instance the compounds class known as growth factors (TGF), are preferably included in layer 7. Compounds of this type are known in the art, for instance from the publications: M. Dettin et al., "Novel Osteoblast-Adhesive Peptides for Dental/Orthopedic Biomaterials", J. *Biomed. Mater. Res. 60*, pp. 466-471, 2002. and J.M. Wozney et al., "Novel regulators of bone formation: molecular clones and activities", *Science*, *242*, pp. 1528-1534, 1988.

Based on the technique named "Peptide Mimicry" peptides will be preferably utilized of the sequence belonging to BMP-2 (BONE MORPHOGENETIC PROTEIN), that are potentially useful in osteoblast growth promotion (peptide growth factors).

Other drugs and biopolymers are contained in or form the outermost layer 8. These drugs have a protection action (for instance, antibiotics), and an "attraction" and bone growth stimulation action; they are microcapsulated or gel-blended with biopolymers. Biopolymers are essentially constituted of cellular adhesion factors; examples of adhesion factors include bioactive peptide sequences such as those containing the arginine-glycine-aspartic acid sequence (RGD), such as for instance the PepTide Coating product (Tweden et al., "Accelerated healing of cardiovascular textiles promoted by an RGD peptide", *J*. *Heart Valve Dis*., 4 luglio 1995, Suppl. 1 S90-7), those containing the GREDVY sequence (Holt et al., "Endothelial cell binding to Dacron Modified with polyethylene oxide and peptide", *ASAIO J*., luglio-settembre 1994; 50(3) : M858-63), or the KGDN sequences (J.W.

Smith et al., *J*. *Biol. Chem*., dicembre 1994, vol. 269, n. 52, pp. 32788-32795) and SWIGLR (Yokosaki et al., *J*. *Blol. Chem*., 17 dicembre 1999, 274(51), pp. 36328-34).

Figure 2 shows an enlarged cavity provided with layers according to the invention. The thickness of layer 7 of hydroxyapathite is about 5-50 microns, while the thickness of the layer of drugs and biopolymers 8 changes according to the application method of the same; in fact, layer 8 may also be applied by dipping or swabbing on the prosthesis at the time of implantation of the same on the bone.

The prostheses of the embodiment shown in Figure 2 is a metal prosthesis. As shown in Figure 2, in this preferred embodiment a layer 9 is interposed between layer 7 of hydroxyapathite and body 1 from metal of the prosthesis. Layer 9 is constituted of a layer from metal oxide or hydroxide, generally, but not exclusively, oxide or hydroxide of the same metal as the prosthesis, and has, among other things, the purpose of preventing bone tissue from coming directly in touch with the metal, and stopping possible corrosion phenomena of the metal by the biological environment. Layer 9 can be obtained with means known in the art, such as for instance dipping in NaCI (the so-called Kokubo method) or in H₂O₂ (the so-called Osaka method), etc. To this layer there may be associated, according to a preferred embodiment, factors of stimulation of cellular adhesion intended for attracting cell localization on the surface.

The process for the production of the prosthesis with an interface according to the invention preferably includes the shaping of the prosthesis interface, in order to have differentiated surface portions, in particular in order to have areas 6 lower with respect to the rest of surface 5. The reason for such difference has been described previously and may be summarized in the obtainment of portions 5 of the prosthesis surface that are forced against the bone and portions 6 that engage to a limited extent or do not engage at all the bone.

Then, the porous layer 2 is obtained according to one of the aforementioned methods, but preferably by mechanical "working" using laser or electron beam or other means as specified above. The cavities are made in a different way on different areas of the surface or the prosthesis interface; in particular, in zone 5 the cavities will have an inclination (Fig. 3) that facilitates the introduction of the prosthesis when it is forced in the housing previously obtained in the bone, while in zone 6 the inclination may be null or opposite to the one of zone 5. There may be obtained different cavities also in a same area, for instance by alternating greater or smaller widths L and/or depth P.

If the prosthesis is a metal prosthesis, the surface of layer 2 so obtained is preferably oxidized or hydroxylated in order to obtain the layer of metal oxide or hydroxide 9, and possibly have additional factors of stimulation of cellular adhesion.

On layer 9, if present, or directly on layer 2, layer 7 is laid of calcium phosphate, preferably hydroxyapathite, possibly enriched with mineral substances, drugs and growth factors. Methods for obtaining layer 7 are known in the art, and include for instance, the precipitation of hydroxyapathite from a solution of simulated body fluid (SBF).

Lastly, layer 8 of drugs and adhesion factors is applied. As mentioned, this layer can be applied directly before the implantation of the prosthesis in the bone by dipping or swabbing and like means.

## Claims

1. A bone prosthesis **characterized in that** its portion of interface with the bone comprises a porous layer (2) and at least an osteoinductive layer containing one or more chemical compositions that can stimulate and promote the growth of bone tissue.

2. The prosthesis according to claim 1, wherein said porous layer comprises cavities (3, 4) having prefixed and reproducible dimensions and arrangement.

3. The prosthesis according to claim 1 or 2, wherein said porous layer is a metal layer and has a surface comprising a layer (9) of metal oxide or hydroxide.

4. The prosthesis according to claim 3, wherein adhesion and cell growth stimulation factors are added to said layer of metal oxide or hydroxide.

5. The prosthesis according to any of the preceding claims further comprising a first layer (7) containing a compound selected from hydroxyapathite and like calcium phosphates, and a second layer (8) comprising drugs and biopolymers.

6. The prosthesis according to claim 5, wherein said first layer (7) comprises also chemical and biochemical substances and compounds selected from Ca, Mg and P salts, low release drugs, growth factors, and mixtures thereof.

7. The prosthesis according to claim 5 or 6, wherein said second layer comprises chemical and biochemical compositions selected from antibiotics and protection drugs, cellular adhesion factors, and mixes thereof.

8. A process of producing a prosthesis according to any of the preceding claims, **characterized in that** it comprises the following steps:
providing a porous layer on the interface surface between the prosthesis and the bone;
providing at least an osteoinductive layer containing one or more chemical compositions that can stimulate and promote bone tissue growth on said porous layer.

9. The process according to claim 8, wherein said porous layer is obtained by mechanical working of a plurality of cavities (3, 4) having prefixed dimensions (L, P) and arrangement (D, α).

10. The process according to claim 8 or 9, wherein said prosthesis is a metal prosthesis, **characterized in that** it also comprises the step of oxidizing said metal porous surface and of adding adhesion factors and cellular growth stimulation factors to said oxidized porous surface before providing said osteoinductive layer.

11. The process according to any of claims 8 to 10, further comprising the steps of providing on said porous layer a first osteoinductive hydroxyapathite-containing layer and a second osteoinductive layer comprising drugs and adhesion compounds.

12. The process according to claim 11, wherein said second layer is applied when implanting the said prosthesis.

13. The process according to any of claims 8-12, wherein said prosthesis is so shaped as to have surface areas lower than the rest of the surface, and in that different cavities are made in different areas.
